# EUROPEAN PATENT SPECIFICATION

(11) **EP 1 675 809 B1**
(45) Date of publication and mention of the grant of the patent: **03.06.2015**
(21) Application number: 04788992.8
(22) Date of filing: 24.09.2004
(51) Int. Cl.: C07C 5/25

(54) **PROCESS FOR ISOMERIZATION OF ALPHA OLEFINS**
VERFAHREN ZUR ISOMIERUNG VON ALPHA-OLEFINEN
PROCEDE POUR L'ISOMERISATION D'ALPHA-OLEFINES

(30) Priority: 26.09.2003 US 506312 P
(43) Date of publication of application: 05.07.2006
(73) Proprietor: Shell Internationale Research Maatschappij B.V., 2596 HR Den Haag (NL)
(72) Inventor: BROWN, David, Stephen, Hercules, CA 94547 (US); DOLL, Michael, Joseph, Katy, TX 77449 (US)
(86) International application number: PCT/US2004/031341
(87) International publication number: WO 2005/031066

(56) References cited:
- EP-A- 0 609 879
- US-A- 4 514 229
- US-A- 4 749 819
- US-A1- 2003 135 080
- US-A1- 2003 144 153
- DATABASE CAPLUS CHEMICAL ABSTRACTS SERVICE, COLUMBUS, OHIO, US; 1987, ELISEEV, N.A: "Equilibria of dehydrogenation of higher n-paraffins to monoolefins" XP002321330 retrieved from STN Database accession no. 1998:454030 -& ELISEEV, N.A.: "Equilibria of dehydrogenation of higher paraffins to monoolefins" IZVESTIYA VYSSHIKH UCHEBNYKH ZAVEDENII KHIMIYA I KHIMICHESKAYA TEKNOLOGIYA, vol. 30, no. 11, 1987, pages 36-39, XP009045266
- DATABASE CAPLUS CHEMICAL ABSTRACTS SERVICE, COLUMBUS, OHIO, US; ELISEEV N.A.: "Thermodynamic study of dehydrogenation of higher n-paraffins to monoolefines" XP002321331 retrieved from STN Database accession no. 1991:206227 -& NEFTEKHIMIYA, vol. 31, no. 1, 1991, pages 31-36, XP009045266

## Description

### Field of the Invention

The invention relates to a method of making an olefinic composition of C₁₆ and/or C₁₈ internal olefins having enhanced C₂-C₅ isomer content.

### Background of the Invention

While alpha olefins have a wide variety of end uses, certain applications, such as paper sizing agents and drilling fluids, additionally require the use of internal olefins. Internal olefins may be produced from the isomerization of alpha olefins. There are a number of catalysts known for the isomerization of alpha olefins to internal olefins. Highly active catalysts are capable of reaching the thermodynamic isomerization limit, thereby giving internal olefins with an equilibrium distribution of double bond isomers. Internal olefins produced in this way are referred to as "fully isomerized."

Isomerized olefins for drilling fluid applications should exhibit a low pour point, low residual alpha olefin content and minimal branching. A low residual alpha olefin content, as well as minimal branching, is especially important because toxicity of the isomers is principally attributable to these factors. The pour point of the olefin mixture is further desired to be as low as possible. WO 00/03961 discloses a process of producing fully isomerized linear alpha olefins having reduced pour points by use of a nickel supported silica/alumina catalyst.

US 4,514,229 relates to a paper sizing composition comprising internal olefins having from 14 to 36 carbon atoms. This document teaches that it is preferable in such an application for the olefins having a double bond at the 2^{nd}, 3^{rd} or 4^{th} carbon position to make up no less than 70 % of the internal olefins.

US 4,749,819 is directed to a prices for the isomerization of a double bond using aluminosilicate, and particularly ferrierite type, catalysts.

The suitability of internal olefins for use in drilling fluids is disclosed in US 2003/144153 and US 2003/135080.

Olefin compositions comprising hexadecene and octadecene olefin compsotions are described in 'Izvestiya Vysshikh Uchebnykh Zavedenii Khimiya I Khimicheskaya Teknologiya' Vol 30, no. 11, 1987, pages 36 to 39; and 'Neftekhimiya' Vol 31, no. 1, 1991, pages 31 to 36, repectively.

EP 0609879 A1 is directed to paper sizing compositions comprising linear internal olefins of which 95% or more contain 18 or more carbon atoms.

Like drilling fluid compositions, paper sizing compositions have been reported to benefit from the incorporation of isomerized olefins. See, for instance, U.S. Patent No. 6,348,132 which teaches an internal olefin sample, wherein the double bonds are near the end of the chain (but not in the alpha position), which renders a superior paper sizing composition compared to fully isomerized products wherein the double bonds of the olefin samples are more equally distributed throughout the carbon chain. It would be desirable to increase the amount of such "lightly isomerized" olefins in the isomerized mixture. In addition, a process of producing the lightly isomerized alpha olefins having lower residual alpha content is desired for use in paper sizing compositions. Such lightly isomerized alpha olefins further show particular promise for use in drilling fluids.

### Summary of the Invention

The invention relates to a process of converting alpha olefins to olefin(s) containing an internal unsaturated bond (internal olefins) while minimizing skeletal isomerization as well as dimerization. In this process, an olefinic composition of C₁₆ and/or C₁₈ alpha olefins isomerizes to a mixture of internal olefin isomers, wherein the isomerized olefin mixture contains less than 40 weight percent of C₁ and C₆-C₈ olefinic isomers and, optionally, olefinic C₉ isomer, the remainder being olefinic C₂-C₅ olefinic isomers. Preferably, the amount of residual alpha olefin content in the isomerized internal olefin mixture is less than 10 weight percent.

The isomerized internal olefin mixture consists of a mixture of double-bond isomers. Preferably, the isomerized internal olefin mixture contains less than 25 weight percent of olefinic C₁ and C₆-C₈ isomers and, optionally, olefinic C₉ isomer, the remainder being C₂-C₅ olefinic isomers.

The isomerized internal olefin mixture is prepared by isomerizing C₁₆ and/or C₁₈ alpha olefins in the presence of a solid acid silica-alumina catalyst wherein the weight ratio of silica to alumina in the solid acid silica-alumina catalyst is from 45:55 to 55:45.

The olefinic composition of C₁₆ and/or C₁₈ alpha olefins is preferably passed through a bed of the catalyst in a single pass or recycle mode. The operating temperature of the bed is maintained at 70° to 140° C.

Such isomerized internal olefin mixtures have particular applicability as paper sizing compositions and as drilling fluids.

### Detailed Description of the Preferred Embodiments.

In the double bond internal olefin isomer description, the subscript number of the carbon denotes the position of the double bond. For example, "C₂" means the isomer wherein the double bond is between the second and third carbon of the isomer. Further, "C₃" means the isomer wherein the double bond is between the third and fourth carbon of the isomer, etc.

An olefinic composition of C₁₆ and C₁₈ alpha olefins can be isomerized to olefin(s) containing internal unsaturated bonds by contacting the olefinic composition with a solid acid silica-alumina catalyst. The weight ratio of silica to alumina in the solid acid silica-alumina catalyst is from 45:55 to 55:45, preferably 50:50. Decreasing the amount of silica typically leads to a decrease in catalyst activity.

The resulting internal olefinic composition of the process of the invention contains less than 40 weight percent of the C₁ and C₆-C₈ isomers and, when the olefinic composition contains a C₁₈ isomer, the C₉ isomer. Sixty weight percent or greater of the resulting internal olefinic composition contains the C₂-C₅ olefinic isomers. Preferably, the isomerized internal olefinic composition comprises less than 25 weight percent of the C₁ and C₆-C₈ isomers, more preferably, less than 13 weight percent of the C₁ and C₆-C₈ isomers and, optionally, the C₉ isomer and, most preferably, less than 6 weight percent of the C₁ and C₆-C₈ isomers and, optionally, the C₉ isomer. Typically less than 3 percent, more typically less than 1 percent, by weight of olefins is branched during the isomerization.

Further, the resulting internal olefinic composition of the process of the invention contains less than 10 weight percent, more preferably less than 6 weight percent, even more preferably less than 3 weight percent, and most preferably less than 2 weight percent of residual alpha olefin content.

The pour point of the isomerized product, while depending on the specific composition of the feedstream, generally is less than 23 ° F (-5° ° C) but greater than -25 ° C and typically greater than -15° C.

The olefinic composition to be isomerized contains a feedstream of C₁₆, C₁₈ alpha olefins or a mixture thereof. Typically, the feedstream is a mixture on a 1:1 weight basis of C₁₆ and C₁₈ alpha olefins though 40:60 to 60:40 weight ratio is further acceptable. More typically, the feedstream contains between 45 to 70 weight percent of C₁₆ alpha olefin (1-hexadecene) and from 30 to 55 weight percent of C₁₈ linear alpha olefin (1-octadecene). Typically, the feedstream would not contain greater than 4 weight percent of branched olefins.

The double-bond isomerization of the C₁₆ and/or C₁₈ alpha olefin containing olefinic composition is preferably first passed over an adsorption bed containing a molecular sieve adsorbent to remove water. The mixture is then preferably passed over a guardbed or absorption unit containing an alumina-based absorbent to remove certain catalyst poisons. This partially purified feed is then double-bond isomerized over the solid acid silica-alumina catalyst. The operating temperature of the isomerization bed is from 70° to 140° C. This heating step may either be a batch or continuous flow reaction.

The solid acid silica-alumina catalyst may be prepared by precipitating alumina in a silica slurry, followed by firing. In a preferred mode, the catalyst may be prepared by reducing the pH of sodium silicate from its relatively high pH of over 11 to 3.0 by the addition of a strong mineral acid, such as sulfuric acid. This results in the formation of a thick, pasty gel. Once the silica gel has been generated, the pH is adjusted upward to approximately 8.1 by the addition of an aluminate, such as sodium aluminate. Once the pH of 8.1 is reached, precipitation begins. At this point, both the aluminate and aluminum sulfate are added at a rate sufficient to maintain the pH at 8.1. Once all of the aluminum sulfate has been added, more aluminate is added to bring the pH up to approximately 10.3. The high pH is required in order that the sulfate may be washed out of the slurry. The material at this point is a thick slurry. The sulfate and the sodium, introduced with the alumina precursors and sulfuric acid, are then washed out of the slurry by treatment of the slurry with deionized water. The washings preferably occur in stages, with the first taking place at pH 10.3 (sulfate removal). For the second wash, the pH is decreased to approximately 6.0 to facilitate the removal of sodium. Finally, the pH is reduced to about 3.7 to allow removal of the last traces of sodium. After this wash, the pH is increased to 5.0 by the addition of a strong base, such as ammonia. The pH must be raised to enable filtering of the slurry. After the washings are completed, the material is spray dried to yield, as a final powder, amorphous silica-alumina. The BET surface area of the catalyst is typically greater than 400 m²/g with a total pore volume of 0.7 to 0.8 g/ml.

Especially preferred as the solid acid silica-alumina catalyst is the X-600 catalyst, a product of Criterion Catalyst Co. This catalyst is capable of isomerizing either the C₁₆, C₁₈ or a blend of C₁₆/C₁₈ isomers to render linear internal olefins having minimal residual alpha olefin content and an enhanced C₂-C₅ isomer content.

In a preferred mode, prior to being contacted with the solid acid silica-alumina catalyst, the olefinic composition feedstream is passed over a water adsorption bed which, preferably contains a molecular sieve. The adsorbent in this adsorption unit is a molecular sieve, either a 3A, 4A or 13X.

The olefinic composition feedstream is then preferably passed over an absorption unit or guardbed for a time sufficient to remove catalyst poisons, including Lewis bases such as phosphines, from the linear alpha olefin feedstream. The absorption unit or guardbed preferably contains an alumina, most preferably an alumina containing only negligible amounts of calcium or magnesium and do not exhibit microrods in their surface morphology.

In a preferred mode, a 0.8 mm alumina trilobe extrudate is employed which is fairly pure alumina made by combining aqueous solutions of sodium aluminate and aluminum sulfate in ratios sufficient to give a pH of approximately 8.0, at which point precipitation of the alumina occurs. The resulting slurry is then washed to remove undesired ions, primarily sulfate and sodium. The pH is then increased to approximately 10.0 by the addition of sodium aluminate. Sulfate is then removed by washing. The pH is then reduced to approximately 7.0 by the addition of a strong mineral acid, such as nitric acid, and the sodium is then removed in the wash. The material is then spray-dried to give the finished powder. The preparation is done in a batch process; the precipitation requiring about one hour. Such products have a BET surface area of about 210-230 m²/g an average length of 1.5 to about 3.0 mm, an average diameter of about 0.77 to about 0.94 mm and a median pore diameter of about 115 to about 130 Å.

Alternatively, the alumina in the absorption unit may be an alumina trilobe shaped aluminum support, such as KL-5715, available from KataLeuna GmBH Catalysts, Germany. Such products have a BET surface area of about 200 m²/g, an average length of 1.5 to about 3.0 mm, an average diameter of about 0.77 to about 0.94 mm and a median pore diameter of about 138 Å.

The olefinic composition feedstream is then contacted with the solid acid silica-alumina catalyst. The catalyst is activated by heating to a temperature between from 25° to 500° C. in flowing nitrogen at atmospheric pressure. The isomerization is carried out in a reactor operated from between 70° to 140° C. and from 5 to 25 psig to produce the linear internal olefin mixture. No post treatment is required to be conducted.

The isomerization is not permitted to proceed to thermodynamic equilibrium. At thermodynamic equilibrium, full isomerization has occurred resulting in an approximately equal distribution of the C₂-C₈ and, where a C₁₈ serves as the olefinic feedstream, C₉ isomers. Operating conditions are varied to prevent full equilibrium from occurring. Preferably, the isomerization process of the invention proceeds until between from 40 to 60 percent equilibrium has occurred. As such, an equilibrium distribution is not achieved.

The linear internal olefins produced by the isomerization process of the invention are useful as paper sizing compositions as well as fluid drilling compositions.

When used for paper sizing, the isomerized olefinic composition of the process of the invention contains, in combination with the isomerized olefins, at least one paper sizing additive. When used in a paper sizing composition, the isomerized olefinic composition preferably contains less than 25 weight percent of the C₁ and C₆-C₈ isomers and, optionally, the C₉ isomer; the remainder being the C₂-C₅ olefinic isomers. Paper is sized by incorporating into the paper the paper sizing composition containing the isomerized olefinic composition. The term "incorporating" as used herein shall mean that the composition may be incorporated into the paper itself (i.e., the composition may serve as internal sizing agent), or may be applied to the surface of the paper (i.e., the composition serves as surface sizing agent).

The paper sizing composition may further include other materials such as, for example alum, as well as pigments, fillers and other ingredients that may be typically added to paper. The sizing compositions of the process of the invention may also be used in conjunction with other sizing agents so as to obtain additive sizing effects. For instance, cationic agents may be used in conjunction with the isomeric mixture. Such materials are cationic in nature or are capable of ionizing or dissociating in such a manner as to produce one or more cations or other positively charged moieties. Such materials which may be employed as cationic agents in the sizing process include alum, aluminum chloride, long chain fatty amines, sodium aluminate, substituted polyacrylamides, chromic sulfate, animal glue, cationic thermosetting resins and polyamides.

The amount of the sizing composition that may be employed to size paper may vary depending, for example, on the particular sizing composition employed, the particular pulp involved, the specific operating conditions, the contemplated end-use of the paper and the like. Generally, smaller amounts of the sizing compositions may be used initially and, if necessary, increased until the desired sizing effect under the circumstances is reached. Desirable concentrations of the sizing compositions that may be employed in the composition based on the dry weight of the pulp in the finished sheet or web, may range from 0.5 to about 20 pounds per ton.

When used as a drilling fluid, the isomerized olefinic composition contains less than 40 weight percent of the C₁ and C₆-C₈ isomers and, optionally, the C₉ isomer; the remainder being the C₂-C₅ olefinic isomers, most preferably less than 13 weight percent. A wellbore may then be drilled by introducing into the wellbore the drilling fluid containing the isomerized olefinic composition. As used herein "drilling fluid" shall include water-based muds as well as oil-in-water emulsions.

The following non-limiting examples, and comparative demonstrations, bring out the more salient features of the invention. All parts are given in terms of weight units except as may otherwise be indicated.

### EXAMPLES

### Example 1.

X-600 (56.5g) was loaded into a standard fixed bed reactor. Two additional reactors were loaded with equivalent catalyst charges. The catalyst beds were activated at 350° C in flowing air then cooled under flowing nitrogen. For the isomerization, the catalyst beds were brought to 70° C. 1-Hexadecene feed was then started at a flow rate of 78 g/hr. The flow was maintained for 117 hours, after which the run was stopped. The product from each of the reactors was combined to give a single composite sample weighing 26.2 kg. Analysis showed the product contained 2.7% by weight dimer. No increase in branching of the hexadecene was detected. The double bond isomer distribution for this sample is shown in Table I wherein the first number before the carbon length denotes the position of the double bond. For example, "2-C16" means the isomer wherein the double bond is between the second and third carbon of the hexadecene isomer. Further, "3-C16" means the isomer wherein the double bond is between the third and fourth carbon of the hexadecene isomer, etc.

**Table I**

| Example | 1-C16 | 2-C16 | 3-C16 | 4-C16 | 5-C16 | 6-C16 | 7-C16 | 8-C16 |
|---|---|---|---|---|---|---|---|---|
| 1 | 1.9 | 45.9 | 29.3 | 11.4 | 8.4 | 1.4 | 1.1 | 0.6 |
| 2 | 36.1 | 19.9 | 10.9 | 10.2 | 7.2 | 7.0 | 5.9 | 3.0 |

### Example 2. (Comparative)

A standard fixed bed reactor was loaded with 10g of a zeolitic catalyst having a ferrierite isotypic structure, as disclosed in U.S. Patent No. 5,849,960. The catalyst was activated at 350° C under flowing nitrogen. For the isomerization, the catalyst bed was brought to 120° C. 1-Hexadecene feed was then started at a flow rate of 43 g/hr. The flow was maintained for 48 hours, after which the run was stopped. The product, 1.9 kg, was then analyzed. The product contained 5.4% by weight dimer. Branching in the hexadecene was increased from 5.7% (feed) to 7.4%. The double bond isomer distribution for this sample is shown in Table I. Table I clearly shows that X-600 is far more effective at reducing the alpha olefin content while not moving the double bonds deeply into the chain.

### Example 3.

X-600 (13.9g) was loaded in a fixed bed, recycle batch reactor. 1-Hexadecene (250g) was loaded into the feed reservoir of the reactor. The catalyst was activated at 100° C in flowing nitrogen for 14 hours. The reactor (catalyst bed and feed reservoir) was heated to 120° C, and olefin was flowed over the catalyst bed at a rate of 500 g/min in recycle mode. Samples were taken periodically, and isomer distributions from three samples are shown in Table II. This example illustrates how the isomer distribution can be controlled by batch time in a recycle process.

**Table II**

| Time | 1-C16 | 2-C16 | 3-C16 | 4-C16 | 5-C16 | 6-C16 | 7-C16 | 8-C16 |
|---|---|---|---|---|---|---|---|---|
| 1 hour | 8.9 | 54.1 | 24.4 | 6.8 | 4.3 | 0.7 | 0.5 | 0.3 |
| 2 hours | 1.8 | 44.2 | 29.2 | 12.2 | 9.2 | 1.5 | 1.2 | 0.6 |
| 4 hours | 1.4 | 32.0 | 24.6 | 16.3 | 14.5 | 5.0 | 4.1 | 2.1 |

### Example 4 (Comparative

The same reaction conditions described in Example 3 were used, except that the catalyst used was the ferrierite zeolite of Example 2. The catalyst loading was 13.9 g. Samples were taken periodically, and isomer distributions from two samples are shown in Table III. While it is possible to control the isomer distribution by batch time, it is not possible to obtain the isomer distributions of Example 3.

**Table III**

| Time | 1-C16 | 2-C16 | 3-C16 | 4-C16 | 5-C16 | 6-C16 | 7-C16 | 8-C16 |
|---|---|---|---|---|---|---|---|---|
| 1 hour | 28.9 | 19.6 | 11.5 | 11.7 | 8.7 | 8.5 | 7.4 | 3.7 |
| 4 hours | 1.8 | 17.2 | 13.4 | 16.2 | 14.2 | 15.4 | 14.7 | 7.1 |

### Example 5

This example illustrates the ability of X-600 to achieve full isomerization. X-600 (30.2 g) was loaded into a standard fixed bed reactor. The catalyst bed was activated at 350° C in flowing air then cooled under flowing nitrogen. For the isomerization, the catalyst bed was brought to 120° C. 1-Hexadecene feed was then started at a flow rate of 40 g/hr. The flow was maintained for 67 hours. The isomer distribution for a sample taken after 17 hours is shown in Table IV.

**Table IV**

| 1-C16 | 2-C16 | 3-C16 | 4-C16 | 5-C16 | 6-C16 | 7-C16 | 8-C16 |
|---|---|---|---|---|---|---|---|
| 1.3 | 15.8 | 14.1 | 17.7 | 16.7 | 14.4 | 13.4 | 6.5 |

## Claims

1. A process for isomerizing an olefinic composition comprising C₁₆ and/or C₁₈ alpha olefins to olefin(s) containing an internal unsaturated bond comprising contacting the olefinic composition with a solid acid silica-alumina catalyst, wherein the weight ratio of silica to alumina is from 45:55 to 55:45, for a time sufficient to render an isomerized internal olefinic composition having less than 40 weight percent of C₁ and C₆-C₈ internal olefinic isomers and, optionally, C₉ internal olefinic isomer, the remainder being C₂-C₅ internal olefinic isomers.

2. The process of claim 1, wherein the amount of C₁ and C₆-C₈ internal olefinic isomers and, optionally, C₉ internal olefinic isomer is less than 25 weight percent.

3. The process of claim 1, wherein the amount of C₁ and C₆-C₈ internal olefinic isomers and, optionally, C₉ internal olefinic isomer is less than 13 weight percent.

4. The process of claim 1, wherein the amount of C₁ and C₆-C₈ internal olefinic isomers and, optionally, C₉ internal olefinic isomer is less than 6 weight percent.

5. The process of any one of the preceding claims, wherein the amount of residual alpha olefin content in the isomerized internal olefinic composition is less than 10 weight percent.

6. The process of any one of the preceding claims, wherein the alpha olefin olefinic composition is passed through a bed containing the solid acid silica-alumina catalyst.

7. The process of claim 6, wherein the operating temperature of the bed is from 70° to 140° C.

8. The process of claims 6 or 7, wherein the alpha olefin olefinic composition, prior to being contacted with the solid acid silica-alumina catalyst, is passed over a water adsorption bed.

9. The process of claim 8, wherein the water adsorption bed contains a molecular sieve.

10. The process of claim 6, wherein the alpha olefin olefinic composition, prior to being contacted with the solid acid silica-alumina catalyst, is passed over an absorption unit or guardbed for a time sufficient to remove catalyst poisons.

11. A process for sizing paper comprising isomerizing an olefinic composition according to the process of any one of claims 1 to 10, combining the thus formed isomerized internal olefin composition with at least one paper sizing additive to form a paper sizing composition and then incorporating said paper sizing composition into the paper.

12. A process of drilling a wellbore comprising isomerizing an olefinic composition according to the process of any one of claims 1 to 10, providing a drilling fluid containing the thus formed isomerized internal olefinic composition and then introducing said olefinic composition into the wellbore.

## Patentansprüche

1. Ein Prozess zur Isomerisierung einer olefinischen Verbindung, umfassend Alpha-Olefine C16 und/oder C18 bis zu Olefin(en), die eine intern ungesättigte Verbindung enthalten, umfassend die Kontaktierung der olefinischen Zusammensetzung mit einem festen Kieselsäure-Tonerde-Katalysator, wobei das Gewichtsverhältnis zwischen Kieselsäure und Tonerde 45:55 bis 55:45 ist, über einen ausreichenden Zeitraum, um eine isomerisierte intern olefinische Zusammensetzung mit weniger als 40 Gewichtsprozent der intern olefinischen Isomere C1 und C6-C8 und, optional, des intern olefinischen Isomers C9 zu erhalten, wobei der Rest aus den intern olefinischen Isomeren C2-C5 besteht.

2. Der Prozess gemäß Anspruch 1, wobei die Menge der intern olefinischen Isomere C1 und C6-C8 und optional des intern olefinischen Isomers C9 weniger als 25 Gewichtsprozent ist.

3. Der Prozess gemäß Anspruch 1, wobei die Menge der intern olefinischen Isomere C1 und C6-C8 und optional des intern olefinischen Isomers C9 weniger als 13 Gewichtsprozent ist.

4. Der Prozess gemäß Anspruch 1, wobei die Menge der intern olefinischen Isomere C1 und C6-C8 und optional des intern olefinischen Isomers C9 weniger als 6 Gewichtsprozent ist.

5. Der Prozess gemäß einem der vorherigen Ansprüche, wobei die Menge des restlichen Gehalts an Alpha-Olefin in der isomerisierten intern olefinischen Zusammensetzungen niedriger als 10 Gewichtsprozent ist

6. Der Prozess gemäß einem der vorherigen Ansprüche, wobei die olefinische Alpha-Olefin-Zusammensetzung durch ein Bett mit dem festen Kieselsäure-Tonerde-Katalysator geleitet wird.

7. Der Prozess gemäß Anspruch 6, wobei die Betriebstemperatur des Betts von 70°C bis 140°C beträgt.

8. Der Prozess gemäß der Ansprüche 6 oder 7, wobei die olefinische Alpha-Olefin-Zusammensetzung vor der Kontaktierung mit dem festen Kieselsäure-Tonerde-Katalysator über ein Wasseradsorptionsbett geleitet wird.

9. Der Prozess gemäß Anspruch 8, wobei das Wasseradsorptionsbett ein molekulares Sieb enthält.

10. Der Prozess gemäß Anspruch 6, wobei die olefinische Alpha-Olefin-Zusammensetzung vor der Kontaktierung mit dem festen Kieselsäure-Tonerde-Katalysator über einen ausreichenden Zeitraum über eine Adsorptionseinheit oder ein Schutzbett geleitet wird, um Katalysatorgifte zu entfernen.

11. Ein Prozess zum Binden von Papier, umfassend die Isomerisierung einer olefinischen Zusammensetzung gemäß dem Prozess eines der Ansprüche 1 bis 10, Verbindung der damit gebildeten isomerisierten intern olefinischen Zusammensetzung mit zumindest einem Papierbinde-Additiv zur Bildung einer Papierbinde-Zusammensetzung und nachfolgende Einbringung der Papierbinde-Zusammensetzung in das Papier.

12. Ein Prozess zum Bohren eines Bohrlochs, umfassend die Isomerisierung einer olefinischen Zusammensetzung gemäß dem Prozess eines der Ansprüche 1 bis 10, zum Bereitstellen einer Bohrflüssigkeit mit der damit gebildeten isomerisierten intern olefinischen Zusammensetzung und die nachfolgende Einbringung der olefinischen Zusammensetzung in das Bohrloch.

## Revendications

1. Procédé d'isomérisation d'une composition oléfinique comprenant des alpha oléfines en C₁₆ et/ou C₁₈ en oléfine(s) contenant une liaison insaturée interne comprenant la mise en contact de la composition oléfinique avec un catalyseur de silice-alumine acide solide, dans lequel le rapport en poids de la silice sur l'alumine est de 45:55 à 55:45, pendant un temps suffisant pour fournir une composition oléfinique interne isomérisée ayant moins de 40% en poids d'isomères oléfiniques internes en C₁ et en C₆-C₈ et éventuellement, d'isomère oléfinique interne en C₉, le reste étant des isomères oléfiniques internes en C₂-C₅.

2. Procédé selon la revendication 1, dans lequel la quantité d'isomères oléfiniques internes en C₁ et en C₆-C₈ et éventuellement, d"isomère oléfinique interne en C₉ est inférieure à 25% en poids.

3. Procédé selon la revendication 1, dans lequel la quantité d'isomères oléfiniques internes en C₁ et en C₆-C₈ et éventuellement, d'isomère oléfinique interne en C₉ est inférieure à 13% en poids.

4. Procédé selon la revendication 1, dans lequel la quantité d'isomères oléfiniques internes en C₁ et en C₆-C₈ et éventuellement, d'isomère oléfinique interne en C₉ est inférieure à 6% en poids.

5. Procédé selon l'une quelconque des revendications précédentes, dans lequel la quantité de teneur en alpha-oléfines résiduelles dans la composition oléfinique interne isomérisée est inférieure à 10% en poids.

6. Procédé selon l'une quelconque des revendications précédentes, dans lequel la composition oléfinique d'alpha-oléfines est passée à travers un lit contenant le catalyseur de silice-alumine acide solide.

7. Procédé selon la revendication 6, dans lequel la température opérationnelle du lit est de 70° à 140°C.

8. Procédé selon les revendications 6 ou 7, dans lequel, avant d'être mise en contact avec le catalyseur de silice-alumine acide solide, la composition oléfinique d'alpha-oléfines est passée au-dessus d'un lit d'adsorption d'eau.

9. Procédé selon la revendication 8, dans lequel le lit d'adsorption d'eau contient un tamis moléculaire.

10. Procédé selon la revendication 6, dans lequel, avant d'être mise en contact avec le catalyseur de silice-alumine acide solide, la composition oléfinique d'alpha-oléfines est passée au-dessus d'une unité d'absorption ou d'un lit de garde pendant un temps suffisant pour éliminer les poisons catalytiques.

11. Procédé d'encollage de papier comprenant l'isomérisation d'une composition oléfinique en suivant le procédé selon l'une quelconque des revendications 1 à 10, la combinaison de la composition oléfinique interne isomérisée ainsi formée avec au moins un additif d'encollage de papier pour former une composition d'encollage de papier et puis l'incorporation de ladite composition d'encollage de papier au papier.

12. Procédé de forage d'un puits de forage comprenant l'isomérisation d'une composition oléfinique en suivant le procédé selon l'une quelconque des revendications des revendications 1 à 10, la fourniture d'un fluide de forage contenant la composition oléfinique interne isomérisée ainsi formée et puis l'introduction de ladite composition oléfinique dans le puits de forage.
